# EUROPEAN PATENT SPECIFICATION

(11) **EP 3 903 861 B1**
(45) Date of publication and mention of the grant of the patent: **07.06.2023**
(21) Application number: 21170714.6
(22) Date of filing: 27.04.2021
(51) Int. Cl.: A61M 15/00, A61M 11/00, A61M 11/06, B65D 83/00, B05B 1/26

(54) **DEVICE AND KIT FOR DISPENSING A NEBULIZED SUBSTANCE**
VORRICHTUNG UND KIT ZUR ABGABE EINER ZERSTÄUBTEN SUBSTANZ
DISPOSITIF ET KIT POUR DISTRIBUER UNE SUBSTANCE NÉBULISÉE

(30) Priority: 27.04.2020 IT 202000009088
(43) Date of publication of application: 03.11.2021
(73) Proprietor: 3A Health Care S.R.L., 25017 Lonato del Garda BS (IT)
(72) Inventor: ABATE, Simone, 25017 Lonato del Garda Brescia (IT); FRACCAROLI, Davide, 25017 Lonato del Garda Brescia (IT)
(74) Representative: Chimini, Francesco

(56) References cited:
- EP-A2- 3 431 132
- WO-A1-2015/019371
- IT-A1-201800 009 586
- US-A1- 2013 067 656

## Description

The present invention relates to the field of devices for nebulizing a substance in order to perform an aerosol therapy treatment.

A capsule suitable to contain at least one liquid substance for aerosol therapy is disclosed in document IT 2018 0000 9586.

In a very common embodiment, these devices comprise a base body provided with a nozzle adapted to be fluidly connected to a source of compressed air. The base body defines a chamber adapted to receive the substance to be nebulized. A lid closes the chamber and is provided with a dispensing mouth which can be connected to an aerosol therapy mask or other suction accessory.

In order to nebulize the liquid substance contained in the chamber, a nozzle cover, commonly also referred to as a "pisper", is fitted onto the nozzle, which forms with the nozzle at least one suction channel for the substance, which is sucked towards the end narrowing of the nozzle cover, exploiting the Venturi effect produced by the pressurized air passing through the nozzle. The nozzle cover is usually provided with a nebulizer element which extends in the form of a peg just above the end narrowing and which, by mechanical impact, breaks the particles of the substance leaving such a narrowing, causing the nebulization of the substance.

In some embodiments, it is possible to make the action of the nebulized substance even more effective by selecting the dimension of the particles of which the nebulized substance is formed, according to the zone of the respiratory tract to be treated. Thus, for example, to treat the upper respiratory tract, for example the larynx and pharynx, larger particles are used with respect to those which must reach the lower respiratory tract, for example the bronchi and lungs.

To obtain this particle dimension selection, the lid forms a hollow tubular portion, open outwardly so as to suck air from the outside by means of the Venturi effect, the lower end of which faces the nebulizer element. By varying the height of this hollow tubular portion, and therefore the distance thereof with respect to the nebulizer element, it is possible to select the dimension of the particles to be conveyed towards the dispensing mouth. In particular, longer tubular portions, and therefore closer to the formation point of the nebulized substance, allow a smaller particulate to be conveyed towards the dispensing mouth, as the larger particles are unable to bypass the lower end of the tubular portion and, by gravity, they fall to the bottom of the chamber of the base body; shorter tubular portions, on the other hand, therefore more distant from the formation point of the nebulized substance, allow even larger particles to be conveyed towards the dispensing mouth.

It is an object of the present invention to facilitate and speed up the preparation of the dispensing device with the substance to be nebulized.

It is another object of the invention to reduce the number of components forming the dispensing device, thus simplifying the production, maintenance, and storage thereof.

Such objects are achieved by a capsule according to claim 1, a dispensing kit according to claim 14, and a method according to claim 16.

The features and advantages of the present invention will become more comprehensible from the following description of preferred embodiments given by way of nonlimiting examples, in which:

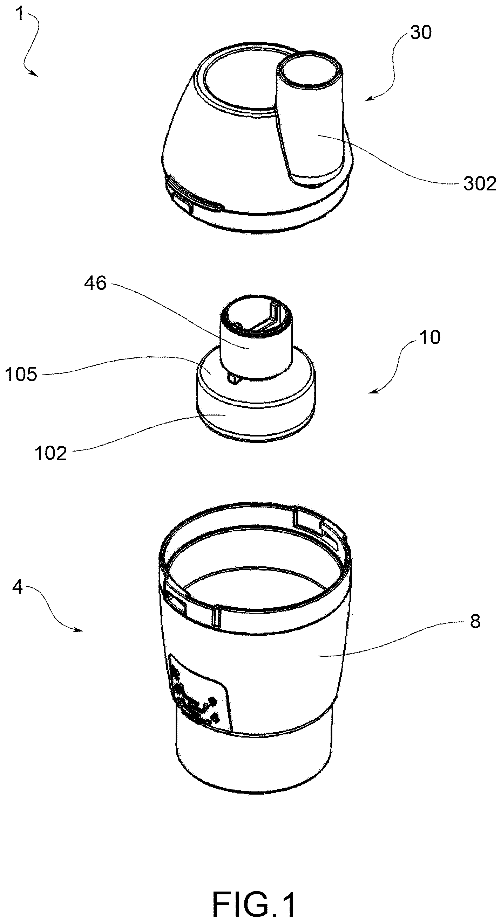

- figure 1 is an exploded perspective view of a dispensing kit in accordance with an embodiment of the present invention;
- figure 2 is an axial section of the dispensing kit;

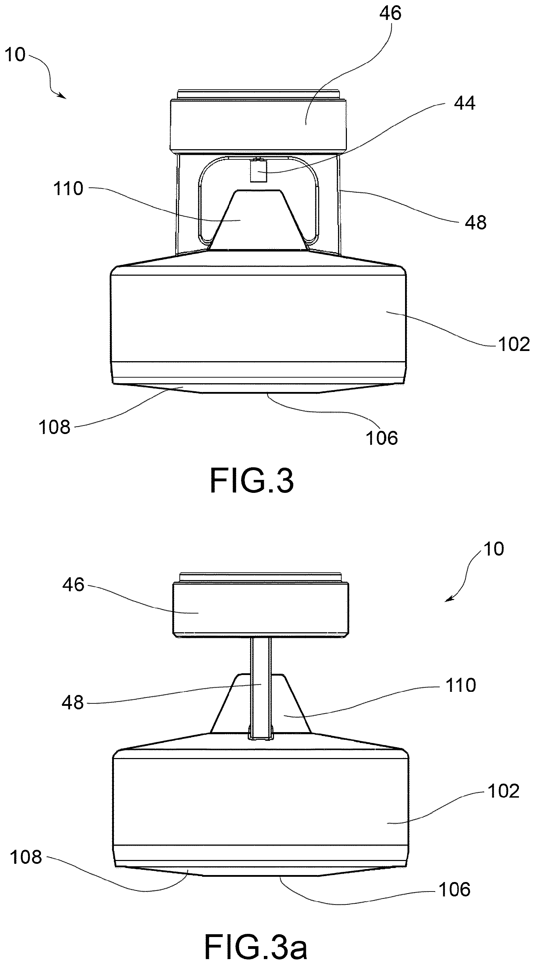

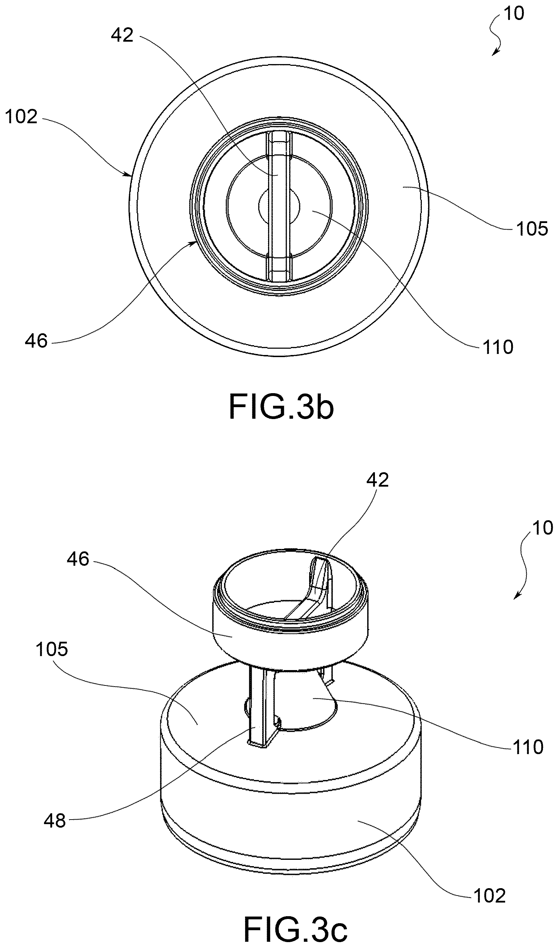

- figures 3, 3a, 3b and 3c are as many views of a capsule according to the invention;
- figures 4 and 4a are two perspective axial sectional views of two capsules according to an embodiment, having cylindrical elements of different heights;
- figure 5 is a perspective axial sectional view of a capsule in an alternative embodiment;
- figures 6 and 6a are an axial section and a perspective axial sectional view, respectively, of a capsule in a further alternative embodiment, with the nebulization assembly separated from the capsule body; and
- figure 7 is a perspective view of a capsule in a further embodiment.

In the continuation of the description and in the accompanying drawings, the elements or parts of elements in common between different embodiments of the invention will be indicated by the same reference numerals.

Furthermore, the terms "upper" and "lower" are used with reference to the dispensing device at rest and positioned on a support surface.

With reference to the aforesaid figures, the reference numeral 1 globally indicates a kit for dispensing a nebulized substance, for example a substance for nasal irrigation or aerosol therapy.

The kit 1 comprises a capsule 10 containing the substance in the liquid state and a dispensing device 4, also known by the term "ampoule", adapted to nebulize the substance contained in the capsule 10.

In a general embodiment, the dispensing device 4 comprises a base body 8 provided with a device nozzle 12 adapted to be fluidly connected to a compressed air source (not shown), such as the compressor of a medical device for nasal irrigation or aerosol therapy.

The device nozzle 12 is crossed by an adduction channel 20 for a flow of compressed air directed along a nozzle axis X and extending between a lower end, which is connectable to the compressed air source, for example by means of a connection to an adduction tube of compressed air, and an upper end 14 which forms a dispensing hole for the compressed air flow.

The base body 8 defines a device chamber 16 adapted to receive the medical substance in the liquid phase to be nebulized through the device nozzle 12.

The device chamber 16 is delimited by a bottom wall 18 and a side wall 20. The device nozzle 12 extends from the bottom wall 18.

In an embodiment, the device nozzle 12 crosses the bottom wall 18 and the connection between the nozzle 12 and the adduction tube is obtained below the bottom wall 18.

The dispensing device 1 further comprises a dispenser lid 30 adapted to be coupled to the base body 8 so as to hermetically close the device chamber and provided with a dispensing mouth 302 adapted to convey the nebulized substance to a mask for aerosol therapy or other suction accessory.

The dispenser lid 30 is provided with a tubular portion 304 which, when the dispenser lid 30 is connected to the base body 8, extends coaxially to the device nozzle 12.

The tubular portion 304 is open at the top towards the outside so as to allow a suction of air from the outside by the Venturi effect.

The capsule 10 comprises a capsule body 102 delimiting a closed chamber 104 adapted to contain the substance to be nebulized in the liquid state.

The closed chamber 104 is delimited at the bottom by a bottom capsule wall 106 in which at least a puncturable and/or tearable and/or peelable portion 108 is obtained to allow the substance to flow out of the capsule 10.

The dispensing kit 1 further comprises capsule opening means obtained in the dispensing device 4 and/or in the capsule 10 and adapted to obtain at least one opening in said at least one puncturable and/or tearable and/or peelable portion 108 as a result of a relative movement between the capsule 10 and the base body 8 of the dispensing device 4.

In an embodiment, the relative movement between the capsule 10 and the base body 8 is obtained through an axial translation or a roto-translation of the capsule inside the device chamber 16, for example until at least one portion of the bottom capsule wall 106 abuts the bottom wall 18 of the device chamber 16.

Therefore, the capsule opening means are adapted to obtain one or more passages in the bottom capsule wall 106 for the outflow of the substance contained in the closed capsule chamber 104 into the device chamber 16 for the subsequent nebulization of the substance.

In order to obtain one or more passages in the bottom capsule wall, the opening means can puncture or tear or peel one or more portions of the bottom capsule wall.

In an alternative embodiment, the opening means can also cause a separation or detachment of the bottom capsule wall from the capsule body. Such a separation or detachment can be due to a compression or traction exerted on the bottom capsule wall.

As will be described below, the opening means can be obtained in the bottom wall 18 of the device chamber 16.

In an alternative embodiment, the opening means, for example in the form of drilling points, can be obtained in a yielding bottom of the capsule, created below the bottom capsule wall 106.

For example, when the capsule 10 is pressed against the bottom wall 18 of the device chamber 16, such a yielding bottom is crushed against the bottom capsule wall 106 causing the puncturing or tearing of one or more of the portions thereof. Naturally, the yielding bottom must be provided with openings to allow the passage of the substance from the closed capsule chamber 104 to the device chamber 16.

There is a capsule nozzle 110 in the capsule body adapted to be fitted onto the device nozzle 12 so as to define at least one substance suction channel 122 with the device nozzle for obtaining the suction of the substance by the Venturi effect.

In an embodiment, the capsule body 102 and the capsule nozzle 110 are made integrally in a single body, for example made of plastic material.

In other embodiments, the capsule nozzle 110 and the capsule body 102 could be made separately and subsequently connected to each other, for example by means of shape and/or force coupling or other coupling systems, such as screw, bayonet, etc.

Since the capsule 10 is configured to be overlapping and surround the device nozzle 12, the capsule nozzle 110 crosses the whole capsule body 102 and protrudes, with a conical upper portion thereof, from a top wall 105 of the capsule body. Therefore, the capsule body 102 has a substantially toroidal shape.

Obviously, the capsule body 102 could also have other shapes different than the annular circular one shown in the drawings, for example as a function of the shape of the base body.

In some embodiments, the closed capsule chamber 104 can be divided into two or more compartments to contain respective different substances, for example different drugs, which can be mixed for example upon opening.

Furthermore, the capsule body 102 supports nebulization means 40 adapted to cause a nebulization of the substance leaving the capsule nozzle 110 by means of an impact of the substance with said nebulization means 40.

Since the capsule nozzle 110 protrudes from the top wall 105 of the capsule body 102, the nebulization means 40 are supported outside the capsule body 102.

In an embodiment, the nebulization means extend from the outer surface of the top wall 105.

In an embodiment, the nebulization means 40 comprise a transverse portion 42 extending orthogonally with respect to the nozzle axis X, and a break pin 44 extending from the transverse portion 42, coaxially to the nozzle axis X, so as to break the particles of the substance leaving the capsule nozzle 110.

In an embodiment, the capsule body 102 further supports a cylindrical element 46 extending coaxially around the nebulization means 40.

Therefore, the cylindrical element 46 is also external to the capsule body 102.

As will be described in more detail below, the height of this cylindrical element 46 determines, together with the cylindrical portion 304 of the dispenser lid 30, the dimension of the particulate which is conveyed towards the dispensing mouth.

In an embodiment, the cylindrical element 46 is integral with the nebulization means 40.

For example, the nebulization means 40 are connected to the capsule body 102 by means of at least two parallel support arms 48 extending from the top wall 105 of the capsule body 102, parallel to the capsule nozzle 110. The cylindrical element 46 can be supported by such parallel support arms 48. More precisely, in an embodiment the cylindrical element 46 surrounds the parallel support arms 48 and is connected to the sides of such arms facing outwards.

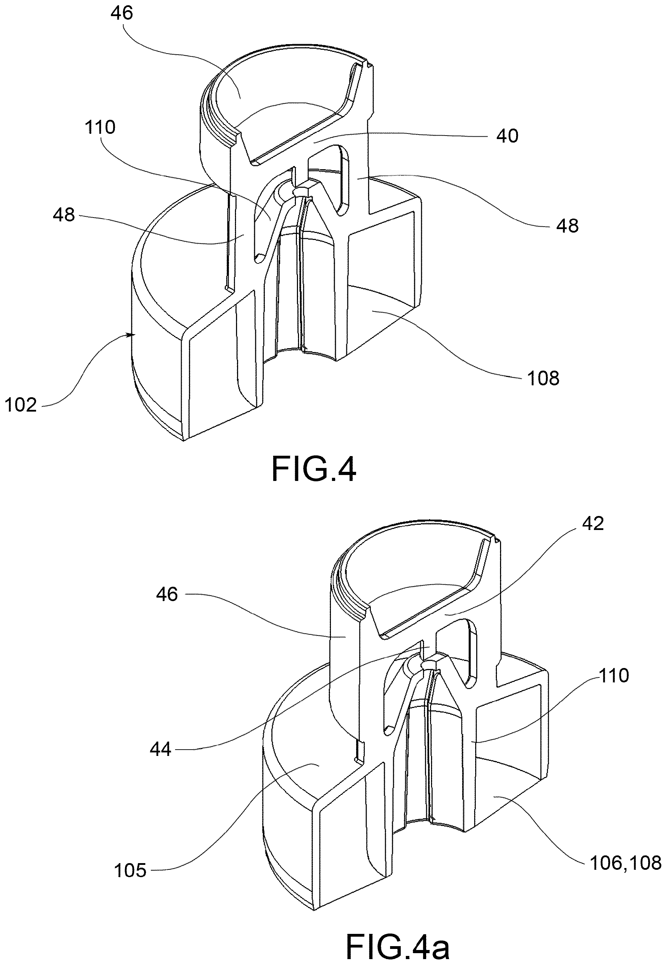

In an embodiment shown in particular in figures 4 and 4a, which show two capsules with cylindrical elements 46 of different length, for the upper and lower respiratory tracts, respectively, the capsule body 102, the nebulization means 40, the support arms 48 and the cylindrical element 46 are made in one piece in a single body made of plastic material.

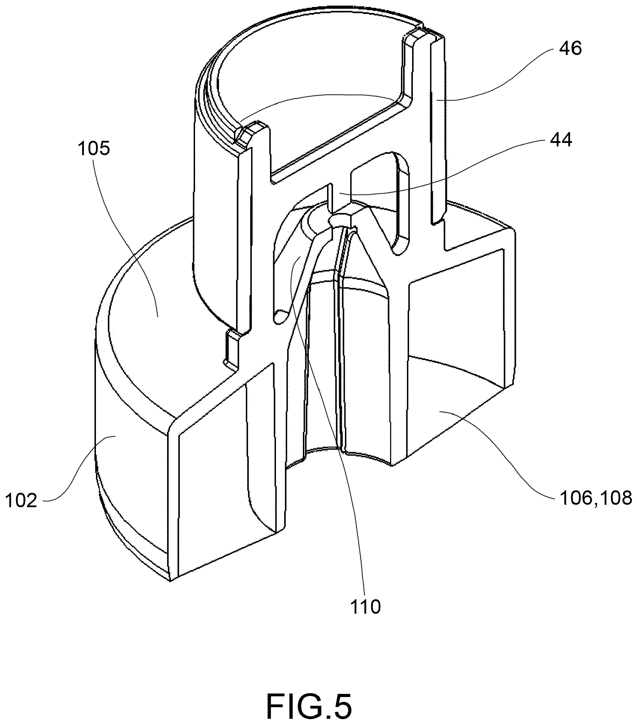

In an alternative embodiment shown in figure 5, which shows the cylindrical element 46 in the elongated version for the lower respiratory tract, the capsule body 102, the nebulization means 40 and the support arms 48 are made in one piece in a single body made of plastic material, while the cylindrical element 46 is removably connected, for example by a snap coupling, to the support arms 48.

In an alternative embodiment, the support arms 48, the nebulization means 40 and the cylindrical element 46 form a nebulization assembly 50 made in one piece in a single body made of plastic material and connected to the capsule body 102.

For example, the nebulization assembly 50 is connected to the capsule body by shape and/or force coupling.

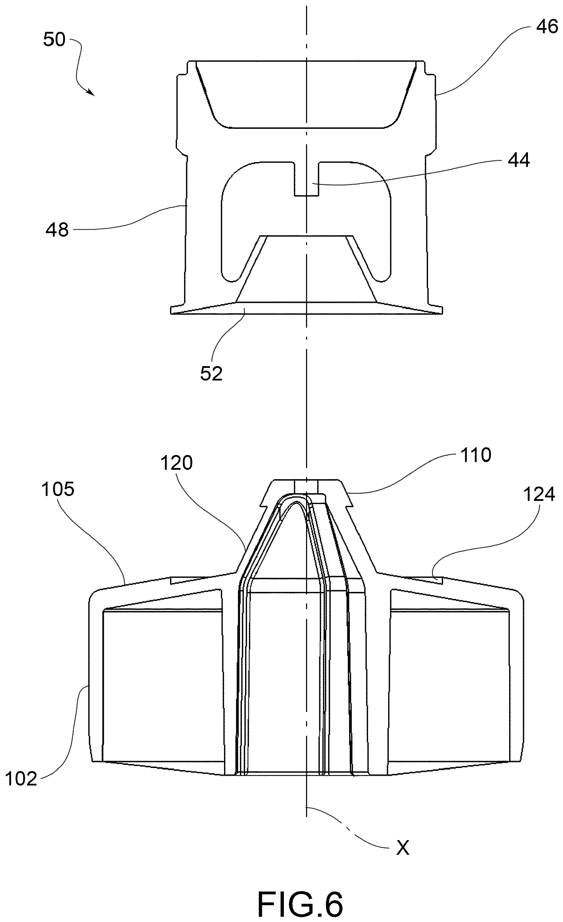

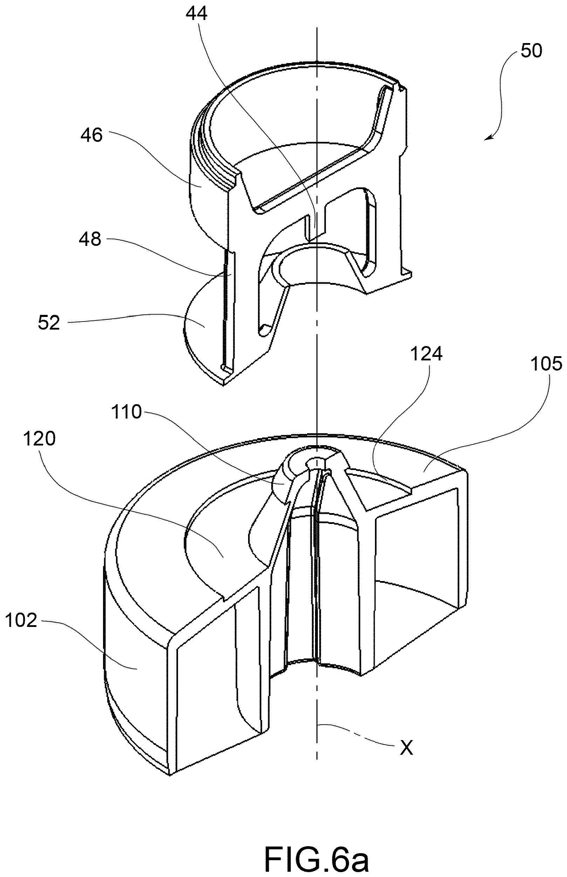

In an embodiment shown in figures 6 and 6a, which show the capsule 10 in the version with cylindrical element 46 of reduced length to obtain a large particulate, there is a lowered zone 120 delimited by an edge 122 which forms an undercut in the top wall 105 of the capsule body 102 and/or in the side wall of a top portion of the capsule nozzle 110, protruding from the top wall 105 of the capsule body. The nebulization assembly 50 comprises a base portion 52 adapted to snap into such a lowered zone 120.

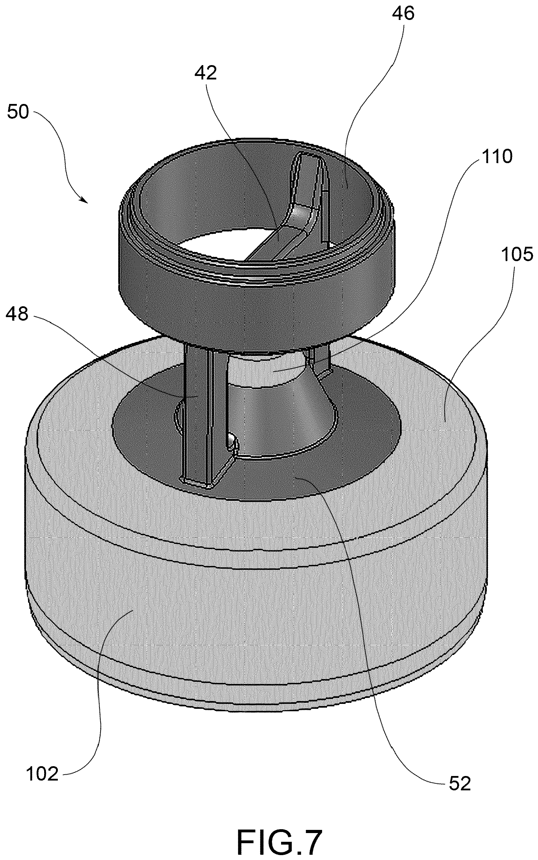

In a further alternative embodiment shown in figure 7, the nebulization assembly 50 is joined to the capsule body 102 by co-molding two different plastic materials, for example TPE and PP.

In an embodiment, the puncturable and/or tearable and/or peelable portion 108 is obtained with a film or a region of smaller thickness than the thickness of the bottom capsule wall.

Returning now to the dispensing device 4, in an embodiment it is provided with capsule opening means obtained in the bottom wall 18.

In an embodiment, the opening means comprise one or more drilling points 182.

In an embodiment, a passage adapted to make the substance flow from the capsule 10 to the device chamber 16 is obtained in each drilling point 182.

In an alternative embodiment, the opening means comprise one or more reliefs adapted to cause a tearing, or a breaking through, of at least a portion of the bottom capsule wall 106 when the bottom capsule wall is pressed against the chamber bottom wall 18 .

In a possible further alternative embodiment, the opening means comprise retaining means adapted to cause a separation of the bottom capsule wall 106 from the capsule body 102 as a result of a relative movement between the capsule 10 and such retaining means.

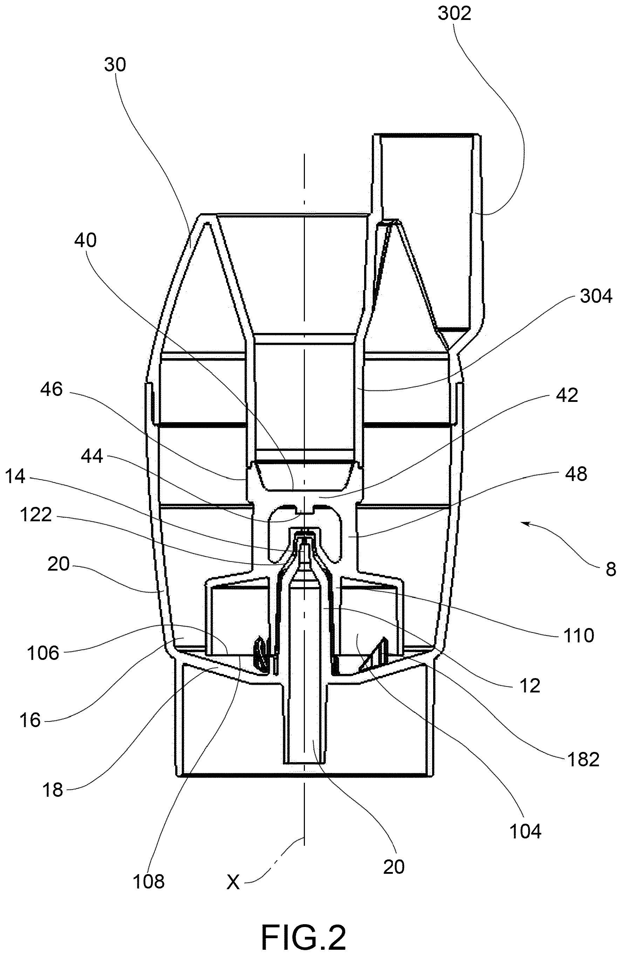

As can be appreciated from figure 2, when the capsule 10 is inserted in the device chamber, the lower end of the tubular portion 304 of the dispenser lid 30 rests on the upper edge of the cylindrical element 46.

Therefore, unlike the dispensing devices according to the known art, it is possible to use a single dispenser lid having a tubular portion of predetermined length, instead of several dispenser lids which differ for tubular portions of different heights. In fact, the cylindrical element of the capsule forms an extension of the tubular portion and the dimension of the particulate can be selected using capsules with cylindrical elements of different heights.

Therefore, the invention also relates to a dispensing kit which comprises at least two capsules 10 having cylindrical elements 46 of different heights, with the upper edge adapted to engage the lower end of the tubular portion 304 of the same dispenser lid 30.

The invention also relates to a method for selecting the dimensions of the particles of the nebulized substance to be dispensed. The method comprises the steps of:
- providing a dispensing kit as described above,
- selecting, from among the capsules of the kit, the capsule having the cylindrical element 46 of a suitable height for allowing only the particles having the desired dimension to flow towards the dispensing mouth.

It is apparent that the dispensing kit, the dispensing device, and the capsule described above allow to achieve the intended objects.

In particular, the user no longer has to worry about carefully dosing the amount of liquid to be introduced into the chamber of the dispensing device. This problem is particularly felt by the elderly, who may have difficulty counting the drops of the substance (especially if it is a drug) to be dispensed from the container thereof to the dispensing device.

While with certain currently used liquid substance containers there is the risk of not being able to pour all the substance into the chamber of the dispensing device, the use of the capsule allows to have the certainty of using all the substance contained therein.

The dispensing device and the assembly thereof are simplified, since the nozzle cover component, or pisper, and the means for the nebulization and selection of the particulate dimensions are obtained directly in the capsule and therefore must not be made as separate components, nor must they be manipulated by the user.

By virtue of the "disposable" solution of the nozzle cover obtained in the capsule body, the substance to be nebulized is less exposed to the risk of contamination due to poor nozzle cover sanitation.

Furthermore, the sealed capsule contains the preestablished amount of substance to be nebulized, unlike some containers (small bottles, vials, etc.) which contain an amount of substance adapted to multiple treatments and which, if not properly reclosed once opened, lead to a contact of the substance with air.

The present invention is defined by the appended claims.

## Claims

1. A capsule (10) adapted to contain at least one liquid substance for aerosol therapy, comprising a capsule body (102) forming a closed chamber (104) adapted to contain the at least one substance, the closed chamber being delimited at the bottom by a bottom capsule wall (106), in which at least one puncturable and/or tearable and/or peelable portion (108) is obtained to allow the substance to flow out of the capsule, wherein a capsule nozzle (110) is obtained in the capsule body, adapted to be fitted onto a device nozzle (12) of a dispensing device so as to define, together with the device nozzle, at least one substance suction channel (122) for the suction of the substance by the Venturi effect, wherein the capsule nozzle (110) crosses the whole capsule body (102) and protrudes, with a conical upper portion thereof, from a top wall (105) of the capsule body, and wherein the capsule body supports nebulization means (40) adapted to cause a nebulization of the substance leaving the capsule nozzle (110) by means of an impact between the substance and said nebulization means (40).

2. A capsule according to claim 1, wherein said nebulization means (40) comprise a transverse portion (42) extending orthogonally with respect to a nozzle axis (X) which is parallel to the direction in which the substance leaves the capsule nozzle, and a break pin (44) extending coaxially to the nozzle axis from said transverse portion so as to break the particles of the substance leaving the capsule nozzle (110).

3. A capsule according to claim 1 or claim 2, wherein the capsule body (102) also supports a cylindrical element (46) extending coaxially around the nebulization means (40).

4. A capsule according to claim 3, wherein the cylindrical element (46) is integral with the nebulization means.

5. A capsule according to claim 4, wherein the nebulization means are connected to the capsule body by means of at least two parallel support arms (48) extending outside the capsule body (102) from the top wall (105) of the capsule body, parallel to the capsule nozzle, and wherein the cylindrical element (46) is supported by said parallel support arms.

6. A capsule according to claim 5, wherein the capsule body, the nebulization means, the support arms, and the cylindrical selection element are made in one piece in a single body made of plastic material.

7. A capsule according to claim 5, wherein the capsule body, the nebulization means, and the support arms are made in one piece in a single body made of plastic material, and wherein the cylindrical selection element is removably connected, for example snap coupled, to the support arms (48).

8. A capsule according to claim 5, wherein the support arms, the nebulization means, and the cylindrical element form a nebulization assembly (50) made in one piece in a single body made of plastic material and connected to the capsule body.

9. A capsule according to the preceding claim, wherein said nebulization assembly is connected to the capsule body by shape and/or force coupling.

10. A capsule according to the preceding claim, wherein a lowered zone (120) is obtained in the top wall of the capsule body and/or in the side wall of a top portion of the capsule nozzle protruding from the top wall of the capsule body, delimited by an edge (122) forming an undercut, and wherein the nebulization assembly comprises a main portion (50) adapted to snap into said lowered zone.

11. A capsule according to claim 8, wherein the nebulization assembly is joined to the capsule body by co-molding two different plastic materials.

12. A capsule according to any one of the preceding claims, wherein the capsule nozzle (110) axially crosses the capsule body (102) so as to give the capsule body a toroidal shape.

13. A capsule according to any one of the preceding claims, wherein said puncturable and/or tearable and/or peelable portion (108) is obtained with a film or a region of smaller thickness than the thickness of the bottom capsule wall.

14. A kit for dispensing a nebulized substance for aerosol therapy, comprising a capsule (10) according to any one of the preceding claims, containing the substance in the liquid state, and a dispensing device (4) comprising:
- a base body (8) provided with a device nozzle (12) adapted to be fluidly connected to a compressed air source, the device nozzle (12) having a hole (14) for dispensing the compressed air,
- the base body (8) defining a device chamber (16) adapted to house the capsule, such that the capsule nozzle (110) overlaps the device nozzle (12), said device chamber being delimited by a bottom chamber wall (18) and by a side wall (20), the device nozzle (12) extending from the bottom wall;
- a dispenser lid (30) adapted to close the base body (8) and provided with a dispensing mouth (302) for the nebulized medical substance, the dispenser lid being provided with a tubular portion (304) extending coaxially to the device nozzle,
wherein, when the capsule is inserted into the device chamber, the lower end of the tubular portion (304) of the dispenser lid rests on the upper edge of the cylindrical element (46),
the kit further comprising capsule opening means (182) obtained in the dispensing device and/or in the capsule and adapted to obtain at least one opening in the puncturable and/or tearable and/or peelable portion as a result of a relative movement between the capsule and the base body of the dispensing device.

15. A kit according to the preceding claim, comprising at least two capsules having cylindrical selection elements of different heights, with the upper edge adapted to engage the lower end of the tubular portion of the same dispenser lid.

16. A method for selecting the dimensions of the particles of the nebulized substance to be dispensed, comprising the steps of:
- providing a dispensing kit according to claim 15,
- selecting, from the capsules in the kit, the capsule having the cylindrical element of a suitable height for allowing only the particles having the desired dimensions to flow towards the dispensing mouth.

## Patentansprüche

1. Kapsel (10), die angepasst ist, zumindest eine flüssige Substanz zur Aerosoltherapie zu enthalten, umfassend einen Kapselkörper (102), der eine geschlossene Kammer (104) bildet, die angepasst ist, die zumindest eine Substanz zu enthalten, wobei die geschlossene Kammer an dem Boden durch eine untere Kapselwand (106) begrenzt ist, in der zumindest ein durchstechbarer und/oder zerreißbarer und/oder abziehbarer Abschnitt (108) enthalten ist, um zu erlauben, dass die Substanz aus der Kapsel herausfließt, wobei eine Kapseldüse (110) in dem Kapselkörper enthalten ist, die angepasst ist, auf eine Vorrichtungsdüse (12) einer Abgabevorrichtung aufgepasst zu werden, um zusammen mit der Vorrichtungsdüse zumindest einen Substanzansaugkanal (122) zum Ansaugen der Substanz durch den Venturi-Effekt zu definieren, wobei die Kapseldüse (110) den gesamten Kapselkörper (102) kreuzt und mit einem konischen oberen Abschnitt davon von einer oberen Wand (105) des Kapselkörpers vorsteht, und wobei der Kapselkörper Vernebelungsmittel (40) stützt bzw. trägt, die angepasst sind, eine Vernebelung der aus der Kapseldüse (110) austretenden Substanz mittels eines Aufpralls bzw. einer Wirkung zwischen der Substanz und den Vernebelungsmitteln (40) zu bewirken.

2. Kapsel nach Anspruch 1, wobei die Vernebelungsmittel (40) einen Querabschnitt (42), der sich orthogonal in Bezug auf eine Düsenachse (X) erstreckt, die parallel zu der Richtung ist, in der die Substanz die Kapseldüse verlässt, und einen Brechstift (44) umfassen, der sich koaxial zu der Düsenachse von dem Querabschnitt erstreckt, um die Teilchen der Substanz zu brechen, die die Kapseldüse (110) verlassen.

3. Kapsel nach Anspruch 1 oder Anspruch 2, wobei der Kapselkörper (102) auch ein zylindrisches Element (46) stützt bzw. trägt, das sich koaxial um die Vernebelungsmittel (40) herum erstreckt.

4. Kapsel nach Anspruch 3, wobei das zylindrische Element (46) einstückig bzw. integral mit den Vernebelungsmitteln ist.

5. Kapsel nach Anspruch 4, wobei die Vernebelungsmittel mit dem Kapselkörper mittels zumindest zweier paralleler Stütz- bzw. Trägerarme (48) verbunden sind, die sich außerhalb des Kapselkörpers von der oberen Wand (105) des Kapselkörpers (102) erstrecken, und zwar parallel zu der Kapseldüse, und wobei das zylindrische Element (46) durch die parallelen Trägerarme getragen ist.

6. Kapsel nach Anspruch 5, wobei der Kapselkörper, die Vernebelungsmittel, die Trägerarme und das zylindrische Auswahlelement in einem Stück in einem einzigen Körper aus Kunststoffmaterial hergestellt sind.

7. Kapsel nach Anspruch 5, wobei der Kapselkörper, die Vernebelungsmittel und die Trägerarme in einem Stück in einem einzigen Körper aus Kunststoffmaterial hergestellt sind, und wobei das zylindrische Auswahlelement mit den Stützarmen (48) lösbar verbunden ist, beispielsweise durch eine Schnappkopplung.

8. Kapsel nach Anspruch 5, wobei die Trägerarme, die Vernebelungsmittel und das zylindrische Element eine Vernebelungsanordnung (50) bilden, die in einem Stück in einem einzigen Körper aus Kunststoffmaterial hergestellt und mit dem Kapselkörper verbunden ist.

9. Kapsel nach dem vorhergehenden Anspruch, wobei die Vernebelungsanordnung mit dem Kapselkörper durch Form- und/oder Kraftkopplung verbunden ist.

10. Kapsel nach dem vorhergehenden Anspruch, wobei eine abgesenkte Zone (120) in der oberen Wand des Kapselkörpers und/oder in der Seitenwand eines oberen Abschnitts der Kapseldüse enthalten ist, der von der oberen Wand des Kapselkörpers vorsteht, und zwar begrenzt durch eine Kante (122), die eine Hinterschneidung bildet, und wobei die Vernebelungsanordnung einen Hauptabschnitt (50) umfasst, der angepasst ist, in die abgesenkte Zone einzuschnappen.

11. Kapsel nach Anspruch 8, wobei die Vernebelungsanordnung durch gemeinsames Formen zweier unterschiedlicher Kunststoffmaterialien mit dem Kapselkörper verbunden ist.

12. Kapsel nach einem der vorhergehenden Ansprüche, wobei die Kapseldüse (110) den Kapselkörper (102) axial kreuzt, um dem Kapselkörper eine Torusform zu verleihen.

13. Kapsel nach einem der vorangehenden Ansprüche, wobei der durchstechbare und/oder zerreißbare und/oder abziehbare Abschnitt (108) mit einem Film oder einem Bereich von geringerer Dicke als der Dicke der unteren Kapselwand enthalten ist.

14. Kit zum Abgeben einer vernebelten Substanz zur Aerosoltherapie, umfassend eine Kapsel (10) nach einem der vorhergehenden Ansprüche, die die Substanz in flüssigem Zustand enthält, und eine Abgabevorrichtung (4), umfassend:
- einen Basiskörper (8), der mit einer Vorrichtungsdüse (12) versehen ist, die angepasst ist, fluidisch mit einer Druckluftquelle verbunden zu sein, wobei die Vorrichtungsdüse (12) ein Loch (14) zum Abgeben der Druckluft aufweist,
- wobei der Basiskörper (8) eine Vorrichtungskammer (16) definiert, die angepasst ist, die Kapsel aufzunehmen, so dass die Kapseldüse (110) die Vorrichtungsdüse (12) überlappt, wobei die Vorrichtungskammer durch eine untere Kammerwand (18) und durch eine Seitenwand (20) begrenzt ist, wobei sich die Vorrichtungsdüse (12) von der unteren Wand erstreckt;
- einen Dispenserdeckel (30), der angepasst ist, den Basiskörper (8) zu verschließen und mit einer Abgabemündung bzw. -Öffnung (302) für die vernebelte medizinische Substanz versehen ist, wobei der Dispenserdeckel mit einem rohrförmigen Abschnitt (304) versehen ist, der sich koaxial zu der Vorrichtungsdüse erstreckt,
wobei, wenn die Kapsel in die Vorrichtungskammer eingesetzt ist, das untere Ende des rohrförmigen Abschnitts (304) des Dispenserdeckels auf der oberen Kante des zylindrischen Elements (46) ruht,
wobei das Kit ferner Kapselöffnungsmittel (182) umfasst, die in der Abgabevorrichtung und/oder in der Kapsel enthalten und angepasst sind, zumindest eine Öffnung in dem durchstechbaren und/oder aufreißbaren und/oder abziehbaren Abschnitt als ein Ergebnis einer relativen Bewegung zwischen der Kapsel und dem Basiskörper der Abgabevorrichtung zu erhalten.

15. Kit nach dem vorangehenden Anspruch, umfassend zumindest zwei Kapseln mit zylindrischen Auswahlelementen unterschiedlicher Höhe, wobei die obere Kante angepasst ist, in das untere Ende des rohrförmigen Abschnitts desselben Dispenserdeckels einzugreifen.

16. Verfahren zum Auswählen der Abmessungen der Partikel der abzugebenden vernebelten Substanz, umfassend die Schritte:
- Bereitstellen eines Abgabekits nach Anspruch 15,
- Auswählen, aus den Kapseln in dem Kit, der Kapsel mit dem zylindrischen Element geeigneter Höhe, um nur den Teilchen mit den gewünschten Abmessungen zu erlauben, in Richtung der Abgabeöffnung zu strömen.

## Revendications

1. Capsule (10) adaptée pour contenir au moins une substance liquide pour une thérapie par aérosol, comprenant un corps de capsule (102) formant une chambre fermée (104) adaptée pour contenir ladite au moins une substance, la chambre fermée étant délimitée au fond par une paroi de capsule inférieure (106) dans laquelle au moins une partie perforable et/ou déchirable et/ou décollable (108) est obtenue pour permettre à la substance de s'écouler hors de la capsule, dans laquelle une buse de capsule (110) est obtenue dans le corps de capsule, adaptée pour être ajustée sur une buse de dispositif (12) d'un dispositif de distribution de manière à définir, conjointement à la buse de dispositif, au moins un canal d'aspiration de substances (122) pour l'aspiration de la substance par l'effet Venturi, dans laquelle la buse de capsule (110) traverse la totalité du corps de capsule (102) et fait saillie, avec une partie supérieure conique de celle-ci, depuis une paroi supérieure (105) du corps de capsule, et dans laquelle le corps de capsule supporte un moyen de nébulisation (40) adapté pour entraîner une nébulisation de la substance quittant la buse de capsule (110) au moyen d'un impact entre la substance et ledit moyen de nébulisation (40).

2. Capsule selon la revendication 1, dans laquelle ledit moyen de nébulisation (40) comprend une partie transversale (42) s'étendant de manière orthogonale par rapport à un axe de buse (X) qui est parallèle à la direction dans laquelle la substance quitte la buse de capsule, et une broche de rupture (44) s'étendant de manière coaxiale à l'axe de buse à partir de ladite partie transversale pour rompre les particules de la substance quittant la buse de capsule (110).

3. Capsule selon la revendication 1 ou la revendication 2, dans laquelle le corps de capsule (102) supporte également un élément cylindrique (46) s'étendant coaxialement autour du moyen de nébulisation (40).

4. Capsule selon la revendication 3, dans laquelle l'élément cylindrique (46) fait partie intégrante du moyen de nébulisation.

5. Capsule selon la revendication 4, dans laquelle le moyen de nébulisation est raccordé au corps de capsule au moyen d'au moins deux bras de support parallèles (48) s'étendant vers l'extérieur du corps de capsule (102) à partir de la paroi supérieure (105) du corps de capsule, parallèle à la buse de capsule, et dans laquelle l'élément cylindrique (46) est supporté par lesdits bras de support parallèles.

6. Capsule selon la revendication 5, dans laquelle le corps de capsule, le moyen de nébulisation, les bras de support, et l'élément de sélection cylindrique sont formés en une seule pièce dans un corps unique constitué de matière plastique.

7. Capsule selon la revendication 5, dans laquelle le corps de capsule, le moyen de nébulisation, et les bras de support sont formés en une seule pièce dans un corps unique constitué de matière plastique, et dans laquelle l'élément de sélection cylindrique est raccordé de manière amovible, par exemple couplé par encliquetage, aux bras de support (48).

8. Capsule selon la revendication 5, dans laquelle les bras de support, le moyen de nébulisation, et l'élément cylindrique forment un ensemble de nébulisation (50) formé en une seule pièce dans un corps unique constitué de matière plastique et raccordé au corps de capsule.

9. Capsule selon la revendication précédente, dans laquelle ledit ensemble de nébulisation est raccordé au corps de capsule par couplage de forme et/ou de force.

10. Capsule selon la revendication précédente, dans laquelle une zone abaissée (120) est obtenue dans la paroi supérieure du corps de capsule et/ou dans la paroi latérale d'une partie supérieure de la buse de capsule faisant saillie à partir de la paroi supérieure du corps de capsule, délimitée par un bord (122) formant une entaille, et dans laquelle l'ensemble de nébulisation comprend une partie principale (50) adaptée pour s'encliqueter dans ladite zone abaissée.

11. Capsule selon la revendication 8, dans laquelle l'ensemble de nébulisation est joint au corps de capsule par co-moulage de deux différentes matières plastiques.

12. Capsule selon l'une quelconque des revendications précédentes, dans laquelle la buse de capsule (110) traverse axialement le corps de capsule (102) pour donner au corps de capsule une forme toroïdale.

13. Capsule selon l'une quelconque des revendications précédentes, dans laquelle ladite partie perforable et/ou déchirable et/ou décollable (108) est obtenue avec un film ou une région d'épaisseur inférieure à l'épaisseur de la paroi de capsule inférieure.

14. Kit de distribution d'une substance nébulisée pour thérapie par aérosol, comprenant une capsule (10) selon l'une quelconque des revendications précédentes, contenant la substance à l'état liquide, et un dispositif de distribution (4) comprenant :
- un corps de base (8) doté d'une buse de dispositif (12) adaptée pour être fluidiquement raccordée à une source d'air comprimé, la buse de dispositif (12) ayant un trou (14) pour distribuer l'air comprimé,
- le corps de base (8) définissant une chambre de dispositif (16) adaptée pour recevoir la capsule, de telle sorte que la buse de capsule (110) chevauche la buse de dispositif (12), ladite chambre de dispositif étant délimitée par une paroi de chambre inférieure (18) et par une paroi latérale (20), la buse de dispositif (12) s'étendant à partir de la paroi inférieure ;
- un couvercle distributeur (30) adapté pour fermer le corps de base (8) et doté d'une bouche de distribution (302) pour la substance médicale nébulisée, le couvercle de distribution étant doté d'une partie tubulaire (304) s'étendant coaxialement avec la buse de dispositif,
dans lequel, lorsque la capsule est insérée dans la chambre de dispositif, l'extrémité inférieure de la partie tubulaire (304) du couvercle distributeur repose sur le bord supérieur de l'élément cylindrique (46),
le kit comprenant en outre un moyen d'ouverture de capsule (182) obtenu dans le dispositif de distribution et/ou dans la capsule et adapté pour obtenir au moins une ouverture dans la partie perforable et/ou déchirable et/ou décollable en résultat d'un mouvement relatif entre la capsule et le corps de base du dispositif de distribution.

15. Kit selon la revendication précédente, comprenant au moins deux capsules ayant des éléments de sélection cylindriques de différentes hauteurs, avec le bord supérieur adapté pour se mettre en prise avec l'extrémité inférieure de la partie tubulaire du même couvercle distributeur.

16. Procédé de sélection des dimensions des particules de la substance nébulisée à distribuer comprenant les étapes de :
- fourniture d'un kit de distribution selon la revendication 15,
- sélection, parmi les capsules dans le kit, de la capsule ayant l'élément cylindrique d'une hauteur adaptée pour permettre qu'uniquement les particules ayant les dimensions souhaitées s'écoulent en direction de l'embouchure de distribution.
